# EUROPÄISCHE PATENTSCHRIFT

(11) **EP 2 824 078 B1**
(45) Veröffentlichungstag und Bekanntmachung des Hinweises auf die Patenterteilung: **18.07.2018**
(21) Anmeldenummer: 13004176.7
(22) Anmeldetag: 23.08.2013
(51) Int. Cl.: C02F 1/42, C02F 1/461, C02F 1/467

(54) **Chlormessung einer Wasseraufbereitungsanlage**
Chlorine measuring of a water treatment system
Mesure du chlore d'une installation de production d'eau

(30) Priorität: 13.07.2013 DE 102013011752
(43) Veröffentlichungstag der Anmeldung: 14.01.2015
(73) Patentinhaber: Vivonic GmbH, 63877 Sailauf (DE)
(72) Erfinder: Völker, Manfred, 63825 Blankenbach (DE)
(74) Vertreter: Flosdorff, Jürgen

(56) Entgegenhaltungen:
- EP-A1- 2 497 750
- WO-A1-2007/096577
- DE-A1- 4 312 600
- DE-A1-102005 049 169
- US-A1- 2001 004 962
- US-B1- 6 174 419

## Beschreibung

Die Erfindung gemäß Anspruch 1 und 6 betrifft ein Fluidsystem zur Qualitäts-, Funktionsüberwachung und/oder Steuerung von physikalisch und chemisch wirkenden Filterstufen einer Wasservorbehandlung für den Betrieb einer Umkehrosmose-, oder einer anderen Wasseraufbereitungs- bzw. Wasserüberwachungsanlage.

Nachteil von Filterstrecken ist die nicht oder nur aufwändig durchzuführende Ferndiagnose von Chlor und Härte bzw. Überwachung des Verschmutzungsgrades von mechanischen Filtern.

Außerdem ist es aus Sicherheitsgründen, insbesondere bei Dialysewasseraufbereitungen, erforderlich, täglich eine aufwändige manuelle Dokumentation der Wasserhärte und oder des Chlorgehaltes durchzuführen, insbesondere um den Nachweis zu führen, dass das toxische Chlor aus der Flüssigkeit durch die eingesetzten Filter entfernt wurde.

Vorhandene Chlorsensoren zur Onlinemessung werden häufig nicht regelmäßig gechlort oder können bei Abwesenheit von Chlor in der Flüssigkeit keine zuverlässigen Messergebnisse liefern.

Um schwer lösbare Salze z.B. Kalzium- und oder Magnesium aus dem Wasser zu entfernen werden häufig Enthärter eingesetzt.
Bei Verwendung von Enthärtern, mit sauren Kationentauscherharzen, sind diese mittels Natriumchloridsolelösung regelmäßig zu regenerieren.
Diese Regeneration wird in der Regel mit Natriumchloridlösung durchgeführt, die in einem sogenannten Solebehälter, in dem Salz in einer vorherbestimmten Flüssigkeitsmenge aufgelöst wird, bereitgestellt ist.
Ein Ausfall der Regenerierung z.B wegen fehlender Natriumchloridsolelösung kann zu gravierenden Verkalkungen der nachfolgenden Anlagen führen.

Darüber hinaus neigen Enthärter wegen der relativ großen Harzvolumen zu mikrobiellem Wachstum mit nachfolgender Kontamination der durchfließenden Flüssigkeit.

Problematisch sind Filterverblockungen, weil der daraus resultierende Austausch von Filtermaterial normalerweise mit einer Betriebsunterbrechung einhergeht.

Ziel der Erfindung ist die Entwicklung einer Aktor-, Sensorsteuerung und einer Software, die es dem Anwender ermöglichen, die Funktionalität einer Anlage per online-Zugriff zu bewerten und auf dieser Basis eine Ferndiagnose über den aktuellen Betriebszustand zu erhalten.

Um den normativen und oder hausinternen Anforderungen zu genügen, können mit der automatischen Registrierung gleichzeitig die erforderlichen Dokumentationsnachweise durch die angeschlossene EDV erbracht werden.

Durch die angestrebte anlagenspezifische Auswertung durch Analyse und Visualisierung der Betriebsparameter wird es möglich, eine azyklische Verteilung der Service-Einsätze und damit eine Verringerung der Servicezahl zu erreichen.

Auf dieser Basis ist ein ökonomisches und ökologischeres Vorgehen möglich, da somit der Einsatz von geschultem Personal vor Ort besser koordiniert und verschleißbedingte Ausfälle gezielt und präventiv vermieden werden können.

Um die vorgenannten Nachteile zu vermeiden, bzw. der Zielsetzung zu entsprechen, werden nach einem Gesichtspunkt der Erfindung vor und nach den Filterstufen mittels geschalteten Ventilen Teilströme zu dem entsprechenden Sensor geführt und durch elektronische Messeinrichtungen ausgewertet. Wobei diese Messeinrichtungen auch integraler Bestandteil von nachfolgenden Anlagen einer Wasseraufbereitung und/oder auch eine Leitwarte sein können. Wobei ein bidirektionaler Betrieb zur Beeinflussung von Aktoren und Sensoren möglich ist.

Mit Vorteil werden mit einem elektronischen Druckaufnehmer unterschiedliche mechanische Filterstufen online auf ihren Verschmutzungsgrad durch Messung der Drücke und Ermittlung der Druckdifferenz überwacht und bei geeigneten Filtern mit entsprechender Rückspülautomatik auch ein automatisches Rückspülprogramm eingeleitet.

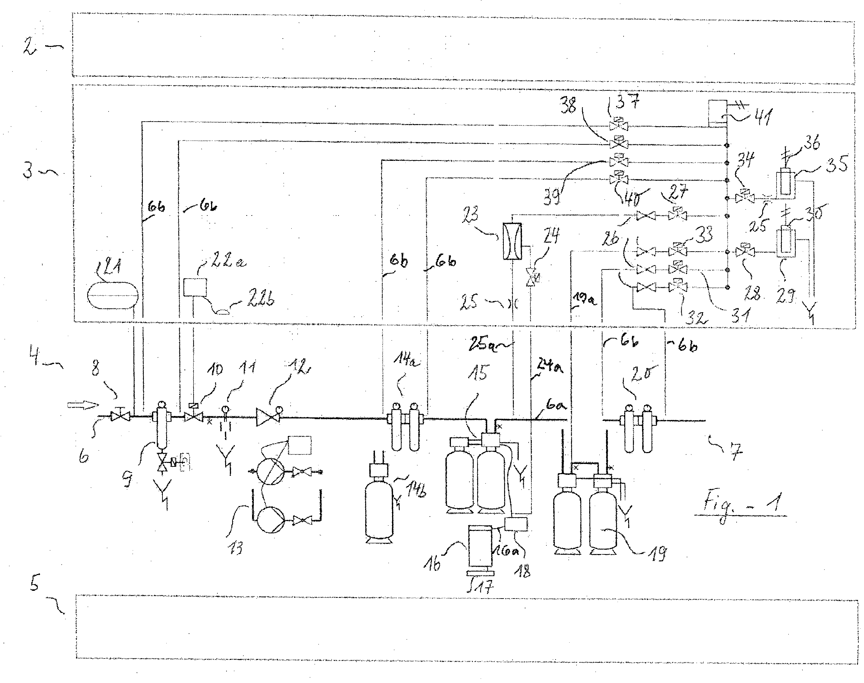

US 2001/00492 A1 offenbart eine Wasserbehandlungsvorrichtung, mit der beispielsweise das Wasser eines Schwimmbeckens behandelt werden kann. Bei der in Figur 1 der Druckschrift dargestellten Ausführungsform zirkuliert das Wasser des Schwimmbeckens zunächst mittels einer Pumpe entlang einer Hauptzirkulationsbahn, in der ein Filter und ein Wärmetauscher angeordnet sind. Von dieser Hauptzirkulationsbahn zweigt ein Wasserbehandlungspfad ab, in dem u.a. ein Restchlorsensor angeordnet ist. Außerdem enthält der Wasserbehandlungspfad als wesentliche Bestandteile einen Lösungstank mit Natriumchlorid und einen elektrolytischen Behälter mit Elektrodenpaaren. Diesem elektrolytischen Behälter wird das abgezweigte Wasser und bei Bedarf eine Natriumchloridlösung aus dem Lösungstank zugeführt. In dem elektrolytischen Behälter wird das Wasser elektrolysiert und sterilisiert, bevor es wieder der Hauptzirkulationsbahn 20 zugeführt wird. US 6,174,419 B1 offenbart eine Vorrichtung zur Erzeugung von elektrolytischem Wasser, bei der eine Anode und eine Kathode in einer Elektrolysezelle periodisch geschaltet werden, um deren Energieverlust zu reduzieren. Außerdem enthält die Vorrichtung einen Konzentrationssensor, um eine Konzentration von Hypochlorsäure zu erfassen.

Die WO 2007/096577A1 offenbart eine Vorrichtung zum Überwachen der Chlorkonzentration im Trinkwasser. Das Chlor desinfiziert das Trinkwasser, wozu es in einer bestimmten Konzentration in dem Trinkwasser enthalten sein muss. Bei zu geringer Konzentration erfüllt es nicht seine Desinfektionsfunktion, während eine Überdosierung den Geschmack beeinträchtigt und zu anderen Gesundheitsschäden führen kann. Die Vorrichtung kann beispielsweise in der Wasserversorgung von Flugzeugen und Gebäuden eingesetzt werden. Die Vorrichtung enthält eine Strömungszelle mit einem Chlorsensor und einem automatischen Kalibrierungssystem zur Erzeugung von freiem Chlor in geringen Konzentrationen sowie Injektionsmittel, um eine Salzlösung bekannter Konzentration in das durchfließende Wasser einzubringen. Mit der Vorrichtung kann eine niedrige Chlorkonzentration in dem Trinkwasser gewährleistet werden.

Gemäß der Erfindung wird ein online messender Chlorsensor eingesetzt, dessen sicherheitsrelevante Funktion erfindungsgemäß überprüft wird, indem dem Sensor elektrolytisch erzeugtes Chlor regelmäßig mit bekannter Konzentration zugeführt wird.

Das Messergebnis wird elektronisch registriert und dokumentiert.
Das Chlor kann aus einer vorhandenen Solelösung hergestellt werden.

Die Funktion des Enthärters, also die Filtration und Reduzierung der schwerlösbaren Calcium und Magnesiumsalze, kann durch einen ionensensitiven Calcium- und oder Magnesiumsensor überwacht werden.

Der Füllstand des Salzsolebehälters, bzw. das Restvolumen der Salze im Solebehälter, ist einfach mittels Waage zu überwachen. Dazu wird der Solebehälter auf ein Konstruktionselement mit Wäagezelle plaziert. Da das konstruktive Untergestell unabhängig von dem verwendeten Solebehälter jederzeit mit Vorteil einzusetzen ist, können auch bereits im Betrieb befindliche Solebehälter mit dieser Überwachungseinrichtung bestückt werden.
Es ist möglich, das Solevolumen direkt- oder als Ampellösung mit Meldungsfarbe anzuzeigen, eine Weiterleitung und Registrierung an eine Leitwarte oder einer nachfolgenden Wasseraufbereitung, die z.B. als RO Anlage ausgebildet sein kann, ist ebenfalls möglich.

Damit kann eine vom Betriebspersonal täglich durchzuführende Inspektion und Dokumentation des Salzvorrates im Solebehälter entfallen.

Ein regelmäßige leichte Chlorierung des Enthärters während der Regenerierung durch elektrolytisch, aus dem Solebehälter des Enthärters erzeugtem Chlor, reduziert das mikrobielle Wachstum im Enthärterharz und sorgt somit für keimfreiere Flüssigkeit.

Figur 1 zeigt eine erfindungsgemäße Vorfiltrationseinheit mit einer mechanischchemischen Filterstufe (4), eine Aktor-Sensor Überwachungseinheit (3), eine zugehörige Auswerteelektronik (2) und eine mögliche, beispielsweise zu einer nachfolgenden Umkehrosmose gehörende Elektronik (5), wobei die Elektronik (2) auch als Leitwarten-Elektronik ausgebildet sein kann und mit Elektronik (5) kommuniziert.

Die mechanisch-chemische Filterstufe (4) ist hinsichtlich der Auswahl der angeordneten Filterstufen nur beispielhaft dargestellt, um die Funktion der erfindungsgemäßen Überwachung zu verdeutlichen.

Die beispielhafte Anordnung beginnt mit dem Wassereingang (6), einem Absperrventil (8), und einem automatisch rückspülbaren Vorfilter (9) mit Abflussventil und Drainageanschluss. Es folgt ein Sicherheitsabsperrventil (10), welches von einem Leckagemelder (22a) mit Flüssigkeitsensor (22b) aktiviert wird.

Weitere Komponenten können ein Rohrtrenner (11) und ein Rückflussverhinderer (12) zur Vermeidung einer Kontamination des Wassereingangs (6) sein.

Bei niedrigen Wasserzulaufdrücken besteht die Möglichkeit der Hinzunahme einer Druckerhöhungseinheit (13). Eine weitere Filterstufe (14) kann sowohl als Kartuschenfilter (14a), Sandfilter (14b), oder als hier nicht dargestellter Hohlfaserfilter im Nano- bzw. Ultraporenbereich ausgebildet sein.

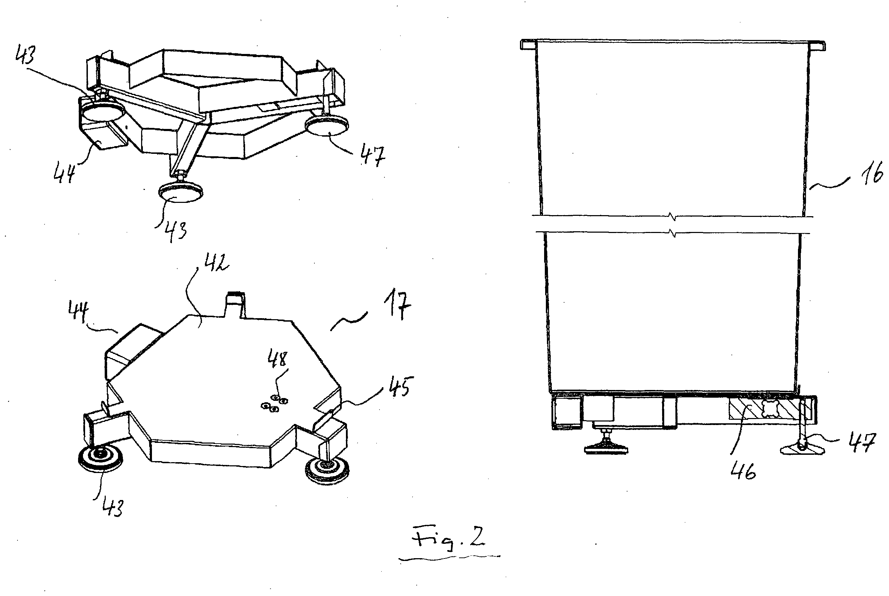

(15) zeigt einen Enthärter, beispielsweise als Doppelenthärter dargestellt, der in der Regel mit starksaurem, kationenhaltigem Harz gefüllt wird, welches bei Erschöpfung regelmäßig mit NaCI-Lösung aus der Salzsoleaufbereitung (16) zu regenerieren ist. Dabei ist es wichtig, den Füllstand des Salzes im Salzsolebehälter (16) zu überwachen. Dies erfolgt mit einer Wäageeinrichtung (17), die als eigenständiges konstruktives Untergestell ausgebildet ist.
Gemäß Figur 2 besteht die Wäageeinrichtung (17) aus einer Wäagezelle (46) deren Signal von Elektronik (44) an der Wäageplattform (42) verstärkt, elektronisch aufbereitet, oder auch von Elektronik (2) als auch von einer evtl. nachfolgenden Elektronik (5) verarbeitet werden kann. Dabei können voreingestellte Gewichtsgrenzwerte des Solebehälters überwacht u. optisch bzw. akustisch zur Anzeige bzw. bei EDV techn. Verarbeitung ferndiagnostiziert werden.
Die Wäagezelle (46) ist an der Wäageplattform (42) mittels Schrauben (48) so befestigt, dass ein Drittel des Sole- bzw. Salzgewichtes auf dem Messfuß (47) lastet. Zur Seitenführung des Solebehälters sind die Seitenbegrenzungen (45) angebracht.

Mittels Elektrolyseeinrichtung (18) kann während des Regenerationsvorgangs des Enthärters (15) chlorhaltige Lösung aus der zur Elektrolysezelle (18) fließenden Salzsole gebildet werden. Dabei versteht es sich, dass die Chlorkonzentration von der Solekonzentration, aber im Wesentlichen von der Höhe der elektrisch zugeführten Leistung zur Elektrolysezelle abhängt. Der mikrobielle Aufwuchs im Enthärterharz wird dadurch stark reduziert.

(19) zeigt einen doppelten Kohlefilter/Dechlorierungseinrichtung, der zur Filtration des Chlors eingesetzt wird.

Filterstufe (20) kann als Feinfilterstufe kleinste Partikel aus dem Filterwasser (7) entfernen, bevor es beispielweise einer Umkehrosmoseanlage oder einer Trinkwasseranlage zugeführt wird.

Die Aktor-Sensor-Einheit (3) kann mit einem elektronischen Wasserzähler (21) zur Registrierung und Meldung der Wasserverbräuche bestückt werden.

Zur Überwachung des Chlorgehalts der zugeführten Flüssigkeit befindet sich in einer Chlorsensorkammer (29) ein Chlorsensor (30), entweder zur Messung des gesamten Chlors oder des freien Chlors.
Die Chlorsensorkammer (29) hat einen Zufluss und einen freien Abfluss. Direkt vor der Sensorkammer befindet sich das Freigabeventil (28).
Üblicherweise kann die zugeführte Flüssigkeit vom Wasserversorger mit Chlor unterschiedlicher Konzentrationen gechlort werden, wobei je nach Hygienezustand unter Umständen zeitweise kein Chloreintrag vorhanden ist.

Insofern ist in diesem Fall keine Aussage über die ordnungsgemäße Funktion des Sensors (30) möglich.

Zur regelmäßigen Überprüfung des Chlorsensors wird das Testventil (27), das Soleansaugventil (24), wie auch das Freigabeventil (28) geöffnet und die Elektrolysezelle (18) eingeschaltet. Dabei wird Sole beziehungsweise Chlorhaltige Lösung in einem ausgewählten Konzentrationsverhältnis aus dem Behälter (16) über das einstellbare Soleansaugventil 24 und die Pumpe (23) angesaugt, mit Flüssigkeit über Flussdrossel (25) vermischt, zur Messkammer (29) weitergeleitet, über Chlorsensor (30) registriert und mit der Elektronik (2) beziehungsweise (5) ausgewertet.

Durch diesen regelmäßigen Test kann die ordnungsgemäße Funktion der Messzelle (30) sichergestellt werden.

Pumpe (23) ist beispielhaft als Venturipumpe dargestellt, andere Pumpentypen sind zur Erreichung der Funktion möglich, wobei in diesem Falle eine Zudosierung der chlorhaltigen Lösung mittels einer nicht dargestellten Pumpe aus Leitung 24a in Leitung 25a erfolgt.

Zur Überwachung der korrekten Kohlefilterfunktion/Dechloriereinrichtung (19) kann zunächst ein Ventil, beispielsweise (40) oder (27) geöffnet werden. Ebenso wird Freigabeventil (28) geöffnet. Falls sich Chlor in der zugeführten Flüssigkeit befindet, wird dies über den vorher verifizierten Chlorsensor (30) registriert.

Danach werden nacheinander die Ventile (33), (31) oder auch (32) geöffnet, ebenso wird das Chlorfreigabeventils (28) geöffnet. So können die Filterstufen des Kohlefilters getestet werden. Registriert der Chlorsensor das Nichtvorhandensein von Chlor ist die Überprüfung des Filters erfolgreich abgeschlossen. Es liegt im Sinne der Erfindung, dass auch diese Messung eigenständig ausgeführt und EDV technisch registriert werden kann.

Zur Überwachung der Filterstufen (9), (14), (20) wird der Druckaufnehmer (41) wahlweise und nacheinander mit den an den Filterstufen herrschenden Drücken über die in Fig. 1 angeordneten Ventile vor oder nach den Filterstufen beaufschlagt.

So findet beispielweise die Überwachung des Druckabfalles der Filterstufe (9) mittels Messung des Eingangsdruckes über Ventil (37) und die Überwachung des Ausgangdruckes durch das Ventil (38) statt.

Äquivalent zu der vorgenannten Messung sind in Figur 1 die Messung der Druckabfälle durch Schaltung der Ventile(39 / 40) für die Filterstufen (14) und der Ventile (31 / 32) für Filterstufe (20) nachzuvollziehen.

Die Ermittlung der Druckabfälle an Enthärtung (15) und Dechlorierung (19) ist durch ein aufeinander folgendes Schalten der Ventile (40, 27, 33, 31) ebenfalls möglich.

Eine atmosphärische Entlastung des Drucksensors (41) generell oder zwischen 2 Messungen kann über Ventil (34) als auch (28) erfolgen.

Durch Messung des Flusses durch Leitung 6 mit Wasseruhr/ Flussmesser (21) oder auch einer entsprechenden Flussmessung in einer nachfolgenden Aufbereitung, können die an den Filtern gemessenen Druckwerte mittels Elektronik (2, 5) auf Standard- bzw. Mittelwerte errechnet und ein Warn-, Austausch-, Spül-, oder Wartungszeitpunkt für voreingestellte Druckdifferenzen prognostiziert werden.

Da es sich bei der Ermittlung der Filter - Druckdifferenzen in der Regel um relative Messungen handelt ist die Verwendung eines einzigen Drucksensors (41), sowohl kostenmäßig, als auch hinsichtlich des Kalibrieraufwandes vorteilhaft.

Normalerweise sind die Wassereingangsdrücke an Leitung (6) z.B. an Filter (9) bekannt, sodass der Drucksensor (41) vor Beginn eines Messzyklus mit einem bekannten Druck beaufschlagt im Rahmen einer Wartung oder Techniker Inspektion zu verifizieren ist.

Eine vorteilhafte Ausgestaltung der Druckmessung ist die Ermittlung von Druckmittelwerten mittels Elektronik (2, 5) an den jeweiligen Filtern (9, 14, 15, 19, 20) indem beispielsweise 50 Messungen zu einem Mittelwert zusammengefasst und über einen beispielhaften Zeitraum von 1000 Betriebsstunden dargestellt werden. Änderungen die auf ein Lebensdauerende des Sensors (41) oder des Verblockens der o.a. Filter zurückzuführen sind, lassen sich damit EDV - technisch erkennen bzw. prognostizieren und fernabfragen.

Zur Überwachung der korrekten Funktion des Enthärters (15) wird zunächst Ventil, (40) geöffnet und dem Calciumsensor (36) durch das geöffnete Ventil (34) Hartwasser über Messkammer (35) zugeführt.
Im Anschluss wird enthärtete Flüssigkeit über Flussdrossel (25), Ventile (27, 34) in die Messkammer (35) zum Ionensensitiven Calciumsensor (36) geleitet.

**Legende**

| | |
|---|---|
| 1. | Vorfiltration mit Sensorpaket |
| 2. | Elektronik Sensorpaket |
| 3. | Aktor- und Sensoreinheit |
| 4. | Vorfiltrationskomponenten |
| 5. | Elektronik Nachfiltration |
| 6. | Wassereingang |
| 7. | Filterwasser |
| 8. | Absperrventil |
| 9. | Rückspülbarer Vorfilter mit Reinigungsventil |
| 10. | Sicherheitsabsperrventil |
| 11. | Rohrtrenner |
| 12. | Rückflussverhinderer |
| 13. | Druckerhöhungseinheit |
| 14. | Feinfilterstufe 2 |
| 15. | Enthärtungsstufe |
| 16. | Salzsoleaufbereitung/ Soletank |
| 17. | Wäageeinheit |
| 18. | Elektrolysezelle |
| 19. | Dechlorierungsstufe/ Kohlefilter |
| 20. | Feinfilterstufe 3 |
| 21. | Wasseruhr/ Flussmesser |
| 22. | Leckagemeldung mit Sensor |
| 23. | Solepumpe |
| 24. | Soleansaugventil |
| 25. | Flussdrossel |
| 26. | Rückflussverhinderung |
| 27. | Chlorsensortestventil / Calciumprüfventil I |
| 28. | Chlorsensorfreigabeventil |
| 29. | Chlorsensorkammer |
| 30. | Chlorsensor |
| 31. | Chlorprüfventil II / Feinfilterstufe 3 Eingangsdruck |
| 32. | Chlorprüfventil III / Feinfilterstufe 3 Ausgangsdruck |
| 33. | Chlorprüfventil I |
| 34. | Calciumsensorfreigabeventil |
| 35. | Calciumsensorkammer |
| 36. | Calciumsensor |
| 37. | Feinfilterstufe 1 Eingangsdruck |
| 38. | Feinfilterstufe 1 Ausgangsdruck |
| 39. | Feinfilterstufe 2 Eingangsdruck |
| 40. | Feinfilterstufe 2 Ausgangsdruck/ Calciumtestventil |
| 41. | Drucksensor |
| 6a | Leitungen |
| 6b | |
| 16a | |
| 19a | |
| 24a | |
| 25a | |
| 42. | Plattform |
| 43. | Einstellbare Füße |
| 44. | Elektronik |
| 45. | Seitenbegrenzung |
| 46. | Wäagezelle |
| 47. | Messfuss |
| 48. | Befestigung Wäagezelle |

## Patentansprüche

1. Verfahren zur Chlorgehaltsüberwachung in einer Wasseraufbereitungsanlage mit wenigstens einer Wasserleitung, einer in die Wasserleitung gesetzten Dechloriereinrichtung (19) und einer Chlorsensoreinrichtung (29, 30),
**dadurch gekennzeichnet,**
**dass** die Chlorsensoreinrichtung einen Online messenden Chlorsensor (30) beinhaltet, dessen sicherheitsrelevante Funktion regelmäßig überprüft wird, indem dem Chlorsensor (30) elektrolytisch erzeugtes Chlor mit bekannter Konzentration in zeitlichen Abständen zugeführt wird und der jeweilige Messwert des Chlorsensors (30) mit einem zugehörigen Sollwert verglichen wird, und das Messergebnis elektronisch registriert und dokumentiert wird,
**dass** die Dechloriereinrichtung (19) Ausgänge hat, welche über Leitungen (19a, 6b) und Schaltventile (31, 33) und ein Freigabeventil (28) mit dem Chlorsensor (30) verbunden sind,
**dass** zur Überwachung der korrekten Funktion der Dechloriereinrichtung (19) zunächst ein Ventil (40) und das Freigabeventil (28) geöffnet werden, damit der vorher verifizierte Chlorsensor (30) eventuell vorhandenes Chlor in der aus der Wasserleitung vor der Dechloriereinrichtung zugeführten Flüssigkeit registriert, und
**dass** danach die Ventile (31, 33) und das Freigabeventil (28) geöffnet werden damit der Chlorsensor (30) registriert, ob sich in dem durch die Leitungen (19a, 6b) zugeführten Wasser Chlor befindet, wobei auch diese Messung eigenständig ausgeführt wird und EDV technisch registriert wird

2. Verfahren nach Anspruch 1,
**dadurch gekennzeichnet,**
**dass** das Chlor aus der Salzlösung eines Salzsolebehälters (16) erzeugt wird.

3. Verfahren nach Anspruch 2,
**dadurch gekennzeichnet,**
**dass** der Salzsolebehälter (16) mit einer Enthärtereinrichtung (15) verbunden ist.

4. Verfahren nach einem der Ansprüche 1 bis 3,
**dadurch gekennzeichnet,**
**dass** das Chlor während des Regenerationsvorgangs der Enthärtereinrichtung (15) dem Chlorsensor (30) zugeführt wird.

5. Verfahren nach einem der Ansprüche 1 bis 4,
**dadurch gekennzeichnet,**
**dass** die Funktion der Enthärtereinrichtung (15) durch einen ionensensitiven Calcium- und/oder Magnesiumsensor überwacht wird.

6. Wasseraufbereitungsanlage mit wenigstens einer Chlorsensoreinrichtung (29, 30) und einer Dechloriereinrichtung (19) in einer Wasserleitung der Wasseraufbereitungsanlage,
**dadurch gekennzeichnet,**
**dass** die Chlorsensoreinrichtung eine Sensorkammer (29) und einen Online messenden Chlorsensor (30) aufweist, und dass der Chlorsensor (30) mit einer Auswerteelektronik (2) verbunden ist, wobei sich ein Freigabeventil (28) als letztes Ventil vor der Sensorkammer (29) befindet,
**dass** die Wasseraufbereitungsanlage eine Salzsoleaufbereitungseinrichtung enthält, die mit der Chlorsensoreinrichtung (29, 30) verbunden ist, wobei in die zugehörige Leitung (16a, 24a) eine Elektrolysezelle (18), und danach eine Pumpe (23) und ein vor dem Freigabeventil (28) geschaltetes Ventil (27) eingeschaltet sind,
**dass** vor der Dechloriereinrichtung (19) eine Leitung (6b) mit einem geschalteten Ventil (40) die Wasserleitung über das Freigabeventil (28) mit dem Sensor verbindet,
**dass** die Dechloriereinrichtung (19) Ausgänge hat, die über Leitungen (19a, 6b), Schaltventile (31, 33) über das Freigabeventil (28) mit der Chlorsensoreinrichtung (30) verbunden sind,
und **dass** die Wasseraufbereitungsanlage eine Aktor-Sensor Überwachungseinheit (3) und eine zugehörige Auswerteelektronik (2) aufweist, die zur Durchführung des Verfahrens nach einem der Ansprüche 1 bis 5 konfiguriert sind.

7. Wasseraufbereitungsanlage nach Anspruch 6,
ferner **gekennzeichnet durch**
eine Enthärtereinrichtung (15), mit der die Salzsoleaufbereitungseinrichtung über eine Salzsoleleitung (16a) verbunden ist,
wobei die Elektrolysezelle (18) in die Salzsoleleitung (16a) eingeschaltet ist, und
zwischen der Elektrolysezelle (18) und der Enthärtereinrichtung (15) eine Chlortestleitung (24a) von der Salzsoleleitung (16a) abzweigt, die zu der wenigstens einen Chlorsensoreinrichtung (29, 30) führt.

8. Wasseraufbereitungsanlage nach Anspruch 6,
**dadurch gekennzeichnet,**
**dass** zu der Pumpe (23) außerdem eine Wasserleitung (25a) führt, in die eine einstellbare Drossel (25) eingeschaltet ist.

9. Wasseraufbereitungsanlage nach Anspruch 8,
**dadurch gekennzeichnet,**
**dass** die Pumpe (23) eine Venturipumpe ist.

10. Wasseraufbereitungsanlage nach Anspruch 8 oder 9,
**dadurch gekennzeichnet,**
**dass** die Zudosierung der chlorhaltigen Lösung mittels Pumpe aus der Leitung (24a) in die Leitung (25a) erfolgt.

11. Wasseraufbereitungsanlage nach einem der Ansprüche 6 bis 10,
**dadurch gekennzeichnet,**
**dass** die Sensorkammer (29) einen Abfluss hat.

12. Wasseraufbereitungsanlage nach einem der Ansprüche 6 bis 11,
**dadurch gekennzeichnet,**
**dass** die Größe der elektrischen Leistung der Elektrolysezelle (18) durch eine Steuereinrichtung (3) einstellbar ist.

13. Wasseraufbereitungsanlage nach einem der Ansprüche 6 bis 12,
**dadurch gekennzeichnet,**
**dass** die Wasseraufbereitungsanlage eine Umkehrosmoseanlage ist.

## Claims

1. A method of monitoring the chlorine content in a wafer treatment system with at least one water conduit, a dechlorination device (19) positioned in the water conduit and a chlorine sensor device (29, 30), **characterised in that** the chlorine sensor device includes a chlorine sensor 30, which measures online, the safety-related function of which is regularly checked by supplying electrolytically produced chlorine of known concentration to the chlorine sensor (30) at time intervals and each measurement value of the chlorine sensor (30) is compared with an associated desired value and the measurement result is electronically registered and recorded, that the dechlorination device (19) has outlets which are connected to the chlorine sensor (30) via conduits (19a, 6b) and switching valves (31, 33) and a release valve (28), that for the purpose of monitoring the correct function of the dechlorination device (19) a valve (40) and the release valve (28) are firstly opened so that the previously verified chlorine sensor (30) registers any chlorine present in the liquid supplied from the water conduit upstream of the dechlorination device, and that the valves (31, 33) and the release valve (28) are then opened so that the chlorine sensor (30) registers whether chlorine is present in the water supplied through the conduits (19a, 6b), wherein also this measurement is performed autonomously and is registered by data processing means.

2. A method as claimed in Claim 1, **characterised in that** the chlorine is produced from the salt solution in a brine container (16).

3. A method as claimed in Claim 2, **characterised in that** the brine container (16) is connected to a water softener device (15).

4. A method as claimed in one of Claims 1 to 3, **characterised in that** the chlorine is supplied to the chlorine sensor (30) during the process of regenerating the wafer softener device (15).

5. A method as claimed in one of Claims 1 to 4, **characterised in that** the function of the water softener device (15) is monitored by an ion-sensitive calcium and/or magnesium sensor.

6. A water treatment system with at least one chlorine sensor device (29, 30) and a dechlorination device (19) in a water conduit of the water treatment system, **characterised in that** the chlorine sensor device includes a sensor chamber (29) and a chlorine sensor (30), which measures online and that the chlorine sensor (30) is connected to an electronic analysis system (2), wherein a release valve (28) is situated as the last valve before the sensor chamber (29), that the water treatment system includes a brine preparation device, which is connected to the chlorine sensor device (29, 30), wherein connected into the associated conduit (16a, 24a) there are an electrolysis cell (18) and after it a pump (2) and a valve (27) connected before the release valve (28), that before the dechlorination device (19) a conduit (66) with a valve (40) connected in it connects the water conduit via the release valve (28) to the sensor, that the dechlorination device (19) has outlets which are connected to the chlorine sensor device (30) via conduits (19a, 6b), switching valves (31, 33) via the release valve (28) and that the water treatment system includes an actuator-sensor monitoring unit (3) and an associated electronic analysis system (27), which are configured to perform the method as claimed in one of Claims 1 to 5.

7. A water treatment system as claimed in Claim 6, further **characterised by** a water softener device (15), to which the brine preparation device is connected via a brine conduit (16a), wherein the electrolysis cell (18) is connected into the brine conduit (16), and branching off from the brine conduit (16a) between the electrolysis cell (16) and the water softener device (15) there is a chlorine testing conduit (24a), which leads to the at least one chlorine sensor device (29, 30).

8. A water treatment system as claimed in Claim 6, **characterised in that** leading to the pump (23) there is also a water conduit (25a), connected into which there is an adjustable throttle (25).

9. A water treatment system as claimed in Claim 8, **characterised in that** the pump (23) is a venturi pump.

10. A water treatment system as claimed in Claim 8 or 9, **characterised in that** the addition of the chlorine-containing solution is effected by means of a pump from the conduit (24a) into the conduit (25a).

11. A water treatment system as claimed in one of Claims 6 to 10, **characterised in that** the sensor chamber (29) has an outlet.

12. A water treatment system as claimed in one of Claims 6 to 11, **characterised in that** the magnitude of the electrical power of the electrolysis cell (18) is adjustable by means of a control device (3).

13. A water treatment system as claimed in one of Claims 1 to 12, **characterised in that** the water treatment system is a reverse osmosis system.

## Revendications

1. Procédé servant à surveiller la teneur en chlore dans une installation de traitement des eaux comprenant au moins une conduite d'eau, un dispositif de déchloration (19) placé dans la conduite d'eau et un dispositif de capteur de chlore (29, 30),
**caractérisé en ce**
**que** le dispositif de capteur de chlore contient un capteur de clore (30) de mesure en ligne, dont le fonctionnement revêtant une importance en matière de sécurité est vérifié régulièrement en ce que du chlore produit de manière électrolytique est amené en une concentration connue à intervalles de temps donnés au capteur de chlore (30) et la valeur de mesure respective du capteur de chlore (30) est comparée à une valeur de consigne associée, et le résultat de mesure est enregistré et consigné de manière électronique,
**que** le dispositif de déchloration (19) est doté de sorties, qui sont raccordées au capteur de chlore (30) par l'intermédiaire de conduites (19a, 6b) et de soupapes de commutation (31, 33) et d'une soupape de déblocage (28),
**qu'**aux fins de la surveillance du bon fonctionnement du dispositif de déchloration (19), une soupape (40) et la soupape de déblocage (28) sont ouvertes dans un premier temps afin que le capteur de chlore (30) vérifié au préalable enregistre le chlore éventuellement présent dans le liquide amené avant le dispositif de déchloration depuis la conduite d'eau, et
**que** par la suite les soupapes (31, 33) et la soupape de déblocage (28) sont ouvertes afin que le capteur de chlore (30) enregistre si du chlore se trouve dans l'eau amenée par les conduites (19a, 6b), dans lequel également ladite mesure est exécutée de manière autonome et est enregistrée par une technique informatique.

2. Procédé selon la revendication 1,
**caractérisé en ce**
**que** le chlore est produit à partir de la solution saline d'un contenant de saumure (16).

3. Procédé selon la revendication 2,
**caractérisé en ce**
**que** le contenant de saumure (16) est raccordé à un dispositif d'adoucissement (15).

4. Procédé selon l'une quelconque des revendications 1 à 3,
**caractérisé en ce**
**que** le chlore est amené au cours de l'opération de régénération du dispositif d'adoucissement (15) au capteur de chlore (30).

5. Procédé selon l'une quelconque des revendications 1 à 4,
**caractérisé en ce**
**que** le fonctionnement du dispositif d'adoucissement (15) est surveillé par un capteur de calcium et/ou de magnésium sensible aux ions.

6. Installation de traitement des eaux comprenant au moins un dispositif de capteur de chlore (29, 30) et un dispositif de déchloration (19) dans une conduite d'eau de l'installation de traitement des eaux,
**caractérisée en ce**
**que** le dispositif de capteur de chlore présente une chambre de capteur (29) et un capteur de chlore (30) de mesure en ligne, et que le capteur de chlore (30) est raccordé à un système électronique d'évaluation (2), dans laquelle une soupape de déblocage (28) se trouve en tant que dernière soupape devant la chambre de capteur (29),
**que** l'installation de traitement des eaux contient un dispositif de traitement de saumure, qui est raccordé au dispositif de capteur de chlore (29, 30), dans laquelle une cellule d'électrolyse (18) et, après, une pompe (23) et une soupape (27) montée avant la soupape de déblocage (28) sont installées dans la conduite (16a, 24a) associée,
**qu'**une conduite (6b) raccorde avec une soupape (40) montée la conduite d'eau au capteur par l'intermédiaire de la soupape de déblocage (28) avant le dispositif de déchloration (19),
**que** le dispositif de déchloration (19) est doté de sorties, qui sont raccordées par l'intermédiaire de conduites (19a, 6b), de soupapes de commutation (31, 33) et par l'intermédiaire de la soupape de déblocage (28), au dispositif de capteur de chlore (30),
et **que** l'installation de traitement des eaux présente une unité de surveillance à actionneur-capteur (3) et un système électronique d'évaluation (2) associé, qui sont configurés aux fins de la mise en oeuvre du procédé selon l'une quelconque des revendications 1 à 5.

7. Installation de traitement des eaux selon la revendication 6,
**caractérisée en outre par**
un dispositif d'adoucissement (15), auquel le dispositif de traitement de saumure est raccordé par l'intermédiaire d'une conduite de saumure (16a),
dans laquelle la cellule d'électrolyse (18) est installée dans la conduite de saumure (16a),
et
une conduite de test de chlore (24a) forme un embranchement avec la conduite de saumure (16a) entre la cellule d'électrolyse (18) et le dispositif d'adoucissement (15), laquelle conduite de saumure mène à l'au moins un dispositif de capteur de chlore (29, 30) .

8. Installation de traitement des eaux selon la revendication 6,
**caractérisée en ce**
**que** par ailleurs une conduite d'eau (25a) mène à la pompe (23), dans laquelle conduite d'eau est monté un système d'étranglement (25) pouvant être réglé.

9. Installation de traitement des eaux selon la revendication 8,
**caractérisée en ce**
**que** la pompe (23) est une pompe Venturi.

10. Installation de traitement des eaux selon la revendication 8 ou 9,
**caractérisée en ce**
**que** l'ajout dosé de la solution contenant du chlore est effectué au moyen de la pompe depuis la conduite (24a) dans la conduite (25a).

11. Installation de traitement des eaux selon l'une quelconque des revendications 6 à 10,
**caractérisée en ce**
**que** la chambre de capteur (29) est dotée d'une évacuation.

12. Installation de traitement des eaux selon l'une quelconque des revendications 6 à 11,
**caractérisée en ce**
**que** l'intensité de la puissance électrique de la cellule d'électrolyse (18) peut être réglée par un dispositif de commande (3).

13. Installation de traitement des eaux selon l'une quelconque des revendications 6 à 12,
**caractérisée en ce**
**que** l'installation de traitement des eaux est une installation à osmose inverse.
